Europäisches Patentamt

European Patent Office

Office européen des brevets

(11) Publication number: **0 007 704**
A2

# (12) EUROPEAN PATENT APPLICATION

(21) Application number: 79301208.9

(22) Date of filing: 22.06.79

(51) Int. Cl.³: **A 61 K 7/06**
**A 61 K 7/08**

(30) Priority: 28.07.78 GB 3146278

(43) Date of publication of application:
06.02.80 Bulletin 80/3

(84) Designated Contracting States:
AT BE DE FR GB IT NL SE

(71) Applicant: BEECHAM GROUP LIMITED
Beecham House Great West Road
Brentford, Middlesex(GB)

(72) Inventor: James, Anthony Hugh
16 Prebend Gardens
Chiswick, London, W.4(GB)

(74) Representative: Walls, Alan James et al,
Beecham Pharmaceuticals Yew Tree Bottom Road
Epsom, Surrey, KT18 5XQ(GB)

(54) Method of preparing a hair conditioning product.

(57) A method of preparing a hair conditioning product comprising incorporating an aqueous suspension of a metal salt of pyridinethione, especially zinc pyridinethione, wherein the salt is suspended using a cathionic suspending agent, in a liquid vehicle comprising a cationic hair conditioning agent.

EP 0 007 704 A2

TITLE MODIFIED
see front page

COMPOSITION FOR TREATING HUMAN HAIR

This invention relates to a process for the preparation of a product for application to human hair after shampooing to impart an attractive appearance and manageability, and to combat dandruff.

It is well known that shampooing removes oils from the hair and leaves it clean but with a rather dull appearance and rather difficult to arrange into a desired style. To counteract these undesirable effects products are available for application to the hair after shampooing to restore an attractive gloss and manageability Such products usually contain cationic substances which are substantive to or coat the individual hair shafts. They are often generically called "conditioners" or "conditioning rinses".

Zinc pyridinethione is a known antidandruff agent, often included in shampoos. During shampooing it is deposited on the hair and scalp, but a large proportion is washed out during rinsing, thereby reducing the effectiveness of the treatment. It would therefore be desirable if zinc pyridinethione (or another metal salt of pyridinethione effective against dandruff) could be applied to the hair after shampooing and rinsing.

One way this might be achieved would be to incorporate the pyridinethione salt in a traditional hair conditioner preparation, to obtain the benefits of good appearance and manageability as well as dandruff control. Zinc pyridinethione can be supplied as a dry powder or an aqueous suspension but the latter is almost always used because of severe toxicity and handling problems with the powder. Zinc pyridinethione suspension is commercially available only using anionic suspension aids. These anionic suspension aids are incompatible with the cationic conditioning agents found in conditioner products.

According to the present invention there is provided a method for the preparation of a composition for treating human hair comprising incorporating an aqueous suspension of a metal salt of pyridinethione wherein said salt is maintained in suspension by means of a cationic suspending agent, in liquid vehicle comprising a cationic conditioning agent.

Since it has not previously been practicable to prepare a hair conditioning product including a salt of pyridinethione, the compositions prepared by the method of this invention are new. Accordingly, in another aspect, the present invention provides a composition for treating human hair comprising a liquid vehicle comprising a cationic conditioning agent, and, suspended therein by means of a cationic suspending agent, a metal salt of pyridinethione.

By the term "conditioning agent" is meant a substance which is substantive to and/or which coats hair fibres to impart a visual gloss and/or manageability to the hair as a whole. Such substances are well known in the hair-care art. They are often quaternary ammonium compounds. Examples of cationic conditioning agents include quaternary ammonium compounds such as stearyl dimethyl benzyl ammonium chloride, distearyl dimethyl ammonium chloride, cetyl trimethyl ammonium chloride, N-stearyl colaminoformyl methyl pyridinium chloride, cetyl ethyl morpholinium 2-etho-sulphate, and mink-amidopropyl dimethyl 2-hydroxyethyl ammonium chloride.

The liquid vehicle comprising the conditioning agent may be an oil-in-water emulsion in which the cationic conditioning agent is present in the oil phase.

- 3 -

Such emulsions can be prepared by the use of conventional emulsifying agents such as cetyl alcohol, cetostearyl alcohol, ethoxylated glycol monostearates and glyceryl monostearate. These emulsifying agents are left behind on the hair shafts after evaporation of water from the oil-in-water emulsion, and also contribute to the overall appearance and manageability of the hair.

Alternatively, the liquid vehicle may be a simple aqueous solution or suspension of conditioning agent, usually opacified by the addition of a water insoluble material.

In the method of the invention, the metal salt of pyridinethione, especially zinc pyridinethione, is incorporated in the liquid vehicle comprising conditioner as an aqueous suspension wherein the suspending agent is cationic. Cationic suspending agents include quaternary derivatives which provide adequate viscosity and appropriate rheological properties to suspend the active ingredient, such as Polymer J R 30 M a high molecular weight quaternised hydroxy ethyl cellulose.

Conventional toiletries additives such as perfume, colour, preservative and the like can be included in the final product at any stage during its preparation.

Typically, the quantities of pyridinethione salt and cationic conditioning agent will be chosen such that from 0.05 to 5.0% w/w of the final product is pyridinethione salt and from 0.1 to 10.0% w/w is cationic conditioning agent.

If an emulsifying agent is used, it will normally be used in amounts of up to about 10.0% w/w, of the final composition.

In use, the compositions will usually be applied to wet hair, usually just after shampooing and rinsing, and can be massaged through the hair with the fingers or combed in.

- 4 -

The following are examples of formulations prepared in accordance with the invention :-

1. Oil in water Creme Rinse

| Formula | % w/w |
|---|---|
| Stearyl Dimethyl Benzyl Ammonium Chloride | 1.00 |
| Cetyl Alcohol | 3.00 |
| White Mineral Oil, | 1.00 |
| Cithrol 4MS | 2.50 |
| Zinc Pyridinethione Suspension | 1.00 |
| Preservatives | qs. |
| Perfume | qs. |
| Dyes | qs. |
| Water | to 100.00 |

2. Oil Free Conditioning Rinse

| Formula | % w/w |
|---|---|
| Cetyl Trimethyl Ammonium Chloride | 1.00 |
| Natrosol 250 HR | 1.20 |
| Genapol TS Pulver | 2.00 |
| Zinc Pyridinethione Suspension | 1.00 |
| Perfume | qs. |
| Dyes | qs. |
| Preservatives | qs. |
| Water | to 100.00 |

Note

pH of product adjusted 6 to 7 using citric acid or caustic soda.

Cithrol 4MS is 4 mole ethoxylated glycol monostearate.

Genapol TS pulver is a fatty acid polyglycol ether, and acts as an opacifying agent.

Natrosol 250 HR is hydroxyethylcellulose.

- 1 -

## Claims

1.  A method for the preparation of a composition for treating human hair comprising incorporating an aqueous suspension of a metal salt of pyridinethione wherein said salt is maintained in suspension by means of a cationic suspending agent, in a liquid vehicle comprising a cationic conditioning agent.

2.  A method as claimed in claim 1 wherein the liquid vehicle comprising the conditioning agent is an oil-in-water emulsion in which the cationic conditioning agent is present in the oil phase.

3.  A method as claimed in claim 1 wherein the liquid vehicle comprising the conditioning agent is an aqueous solution or suspension of cationic conditioning agent.

4.  A method as claimed in any one of the preceding claims wherein the cationic conditioning agent is selected from stearyl dimethyl benzyl ammonium chloride, distearyl dimethyl ammonium chloride, cetyl trimethyl ammonium chloride, N-stearyl colaminoformyl methyl pyridinium chloride, cetyl ethyl morpholinium 2-etho-sulphate, and mink-amidopropyl dimethyl 2-hydroxyethyl ammonium chloride.

5.  A method as claimed in any one of the preceding claims wherein the cationic suspending agent is a quaternised hydroxyethylcellulose.

6.  A method as claimed in any one of the preceding claims wherein the metal salt of pyridinethione is the zinc salt.